# EUROPEAN PATENT APPLICATION

(11) **EP 3 369 367 A1**
(43) Date of publication of application: **05.09.2018**
(21) Application number: 17159178.7
(22) Date of filing: 03.03.2017
(51) Int. Cl.: A61B 5/00, A61B 5/024, A61B 5/0255, A61B 5/0295

(54) **METHOD, DEVICE AND COMPUTER PROGRAM TO MEASURE A BLOOD PULSE-RELATED PARAMETER OF A USER**

(71) Applicant: Digital for Mental Health, 91300 Massy (FR)
(72) Inventor: FOMPEYRINE, Denis, 92350 Le Plessis Robinson (FR); MOULIERAS, Simon, 75015 Paris (FR)
(74) Representative: Cabinet Plasseraud

(57) **Abstract**

The method to measure a blood pulse-related parameter of a user, comprises:
- a measure, for a long period of time, typically over 6 hours, of blood-pulse related data of a user,
- a generation of high-quality data from the blood-pulse related data by filtering out low-quality data from the blood-pulse related data,
- a determination of the blood pulse-related parameter from the high-quality data.

## Description

### FIELD OF THE INVENTION

The present invention is related to methods, devices and computer programs to measure blood-pulse related parameters of users.

### BACKGROUND TO THE INVENTION

With the development of telecommunication systems, there has been a trend for monitoring people by using such systems. Indeed, the more data can be obtained about someone, the more appropriate service can in theory be rendered. A typical example of such monitoring can be found in sales and advertisement, where paper questionnaires about consumption habits were gradually replaced by on-line polls, and even survey of electronic communication.

This trend was also observed in the medical world. In the field of diagnosis, for example, in everyday life, the doctor (or physician) has very little time with the patient in order to observe the patient, and perform a diagnosis based on this information. During the interview, patients do not always express what is necessary for the physician to make the diagnosis. This is particularly true in the context of assessment grids of mental illness, such as DSM-V. The duration of the interview counts for a lot, 20 minutes minimum should be required. Otherwise further evolution of the disease can be very rapid out of the consultation. According to a recent study ("Clinical diagnosis of depression in primary care: a meta-analysis", Mitchell and al., The Lancet, Volume 374, Issue 9690, 22-28 August 2009, p. 609), 50% of primary care physicians (general practitioners) miss the diagnosis of clinical depression.

A better, and in particular, longer, observation of the patient could be useful in order for the doctor to obtain more information about the patient, so as to be able to perform a proper diagnosis. However, for economical reasons, longer observation periods are not possible. Further, some patients may be unwilling to visit their doctor, whereby a diagnosis is not possible.

The above discussion is particularly true in the field of mental health. For physical illnesses, physiological symptoms can be detected to enable a diagnostic. In the field of mental health, however, there might be few visible symptoms of the illness, which are difficult to detect, and/or can be more or less hidden by the patient, especially for primary care physicians, or even specialists in other fields than mental health. Especially, the patient might visit the doctor about a physical illness which may be caused, or at least superimposed to a mental illness. Not even mentioning being able to diagnose a mental illness, the patient's doctor has to decide whether it is necessary for the patient to visit a specialist based on only a few minutes spent with his patient.

Recently, it was proposed in WO 2016/151,135 to determine a pattern index of a human user, representative of the user's likeliness of exhibiting a pattern of mental suffering, based on data from the human user. This invention has been very successful.

Upon further research, it was considered that blood-pulse can enable to provide data which can be useful data for the implementation of WO 2016/151,135.

It is known to measure blood-pulse during a medical test, with specific material used by a trained physicist or nurse. However, within the context of WO 2016/151,135, we are looking to obtain blood-pulse related data outside from the visit of the user to a medical facility.

Some wearable devices claim measuring blood pulse. However, at best, these devices are able to provide information about some average blood pulse during a certain lapse of time. However, this data is not reliable enough to be used in a medical device. For example, it may suffer a lot from motion artifacts from the user, which would prevent using the data for performing any measurement more precise than a rough estimation of the average blood pulse.

### SUMAMRY OF THE INVENTION

Below, we summarize the invention.

According to a first aspect, the invention relates to a method to measure a blood pulse-related parameter of a user, comprising:
- measuring, for a long period of time, typically over 6 hours, blood-pulse related data of a user,
- generating high-quality data from said blood-pulse related data by filtering out low-quality data from said blood-pulse related data,
- determining said blood pulse-related parameter from said high-quality data.

Thus, advantage is taken of the fact that data is acquired for a very long time in order to select the part of the data which is reliable enough for the required purpose. According to the invention, whenever there is a doubt as to the reliability of the data for the required purpose, the data would not be taken into account. Only data estimated to be reliable would be taken into account for further treatments.

According to one embodiment, generating high-quality data comprises :
determining at least one part of said blood-pulse related data comprising user motion artifacts, and
   removing said at least one part from said blood-pulse related data.

According to one embodiment, generating high-quality data comprises :
determining at least two parts of said blood-pulse related data comprising low-quality data,
   when the time duration between said two parts is lower than a predetermined threshold, filtering the part of said blood-pulse related data between said two parts out of said blood-pulse related data.

According to one embodiment, determining the blood-pulse related parameter involves determining a variability of a RR signal of a plethysmogram, where "R" designates the time of a period where the amplitude reaches the maximum for the period, and "RR" designates the time duration between two subsequent Rs.

According to one embodiment, determining the variability of a RR signal of the plethysmogram involves determining a RR signal of the plethysmogram.

According to one embodiment, determining a RR signal of the plethysmogram involves determining Rs from said high-quality data.

According to one embodiment, determining Rs comprises determining local maxima in said high-quality data along time, and selecting, among said local maxima, selected local maxima having values in a pre-determined window.

According to one embodiment, selecting said local maxima involves using a cost function tending to obtain the most regular time duration between adjacent local maxima.

According to one embodiment, determining the variability of a RR signal of the plethysmogram involves determining the variability for each part of the high quality data.

According to one embodiment, determining the variability of a RR signal of the plethysmogram involves determining an evolution over time of a standard deviation of the RR signal within a time window.

According to one embodiment, determining the variability of a RR signal of the plethysmogram involves determining an evolution over time of a parameter of the spectral density of the RR signal within a time window.

According to one embodiment, said parameter of the spectral density of the RR signal involves a ratio of a low frequency spectral density to a high frequency spectral density of the RR signal within said time window.

According to one embodiment, a spectral density of the RR signal is determined using a transform taking into account irregulary-sampled data without interpolation.

According to another aspect, the invention relates to a device to measure a blood pulse-related parameter of a user, comprising:
at least a sensor measuring, for a long period of time, typically over 6 hours, blood-pulse related data of a user,
a processor generating high-quality data from said blood-pulse related data by filtering out low-quality data from said blood-pulse related data,
Wherein said processor is further configured to determine said blood pulse-related parameter from said high-quality data.

According to another aspect, the invention relates to a computer program comprising instructions which, when the program is executed by a computer, cause the computer to carry out the above method.

### BRIEF DESCRIPTION OF THE DRAWINGS

The drawings will now be briefly described.
- Fig. 1 is a schematic view of an embodiment of the invention into a wristband,

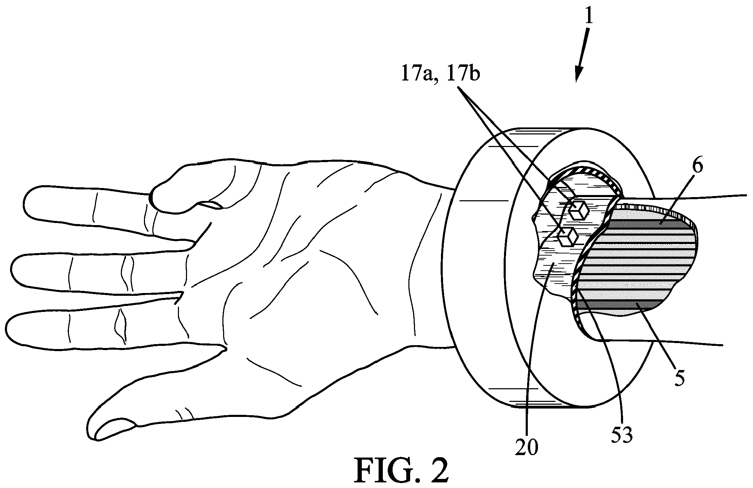

- Fig. 2 is a perspective view of the wristband of Fig. 1,
- Fig. 3 is a general sectional view of the embodiment of Fig. 1,
- Fig. 4 is a view of a detail of Fig. 3,
- Fig. 5 is a time diagram for a time lapse over 13 hours,
- Fig. 6 is a part of the time diagram of Fig. 5, identified as "VI" on Fig. 5,
- Fig. 7 is a time diagram of a RR signal based on that of Fig. 6, and
- Fig. 8 is a schematic diagram of a monitoring device.

In the figures, a given reference numeral designate the same or a similar feature.

A detailed description will now be provided.

### DETAILED DESCRIPTION

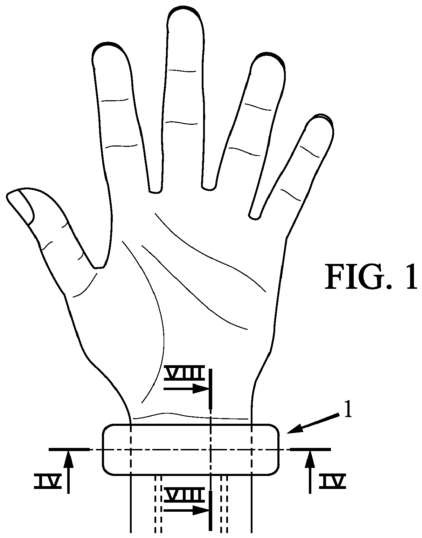

Fig. 1 schematically shows an embodiment of a monitoring device 1 according to the invention. The monitoring device 1 is worn by the user. In the present example, the monitoring device 1 is worn by the user at a wrist. The present description is performed with respect to the left wrist of the user, as shown.

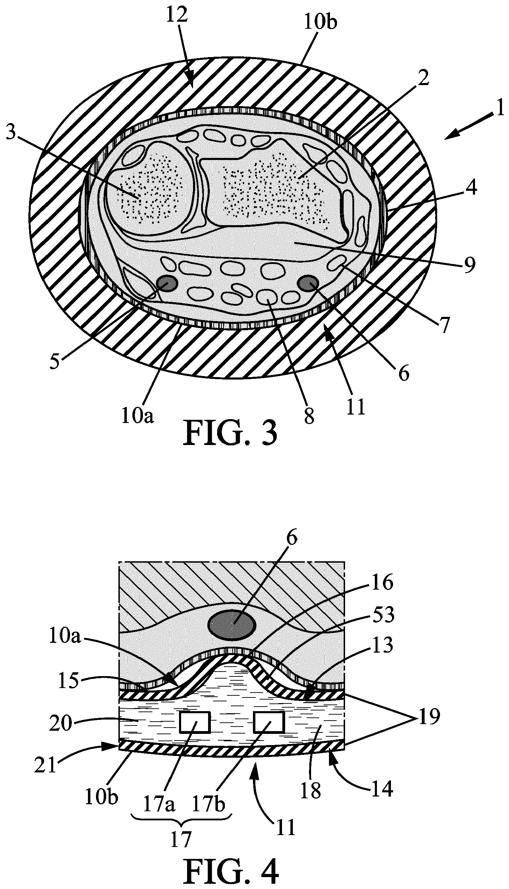

Fig. 3 is a typical sectional view through the left wrist of the user. Fig. 3 is presented with the back of the hand facing upward. The radius 2 and ulna 3 are schematically represented, as well as the skin 4 of the patient. Also shown is the ulnar artery 5, which is close to the ulna 3, and the radial artery 6, which is close to the radius. At the wrist, the radial artery 6 passes between the brachioradialis muscle tendon 7, the flexor carpi radialis muscle tendon 8 and by the pronator quadratus muscle 9, which together define a rigid channel for the radial artery 6.

The monitoring device 1 can be embodied as a band which extends continuously around the whole periphery of the wrist. The device has a radially inner surface 10a and a radially outer surface 10b which is opposite to the radially inner surface 10a. The monitoring device 1 comprises a sensing portion 11 and an attachment portion 12. The attachment portion 12 attaches the monitoring device 1 to the user's limb. In the present case, the attachment portion 12 attaches the monitoring device 1 to the user limb by surrounding in a tight manner the user's limb, in the manner of a wristband. The sensing portion 11 senses the blood pressure. The device will be called a blood pressure monitoring device. This does not mean that the device is able, by itself, to explicitly determine the blood pressure. It could for example determine a blood pressure-related parameter, such as blood pressure variation with time, or the like.

A detailed example of the sensing portion 11 will be provided below with reference to Fig. 4.

On Fig. 4, the sensing portion 11 is presented facing the radial artery 6.

When the monitoring device is fitted on the user's limb, the sensing portion fits tightly the geometry of the user's limb, especially that of the internal hard structures, such as the bones, muscles and tendons. In particular, the sensing portion comprises a flexible film 53, which is provided radially inward. Hence, the radially inner surface of this film 53 forms the radially inner surface 10a of the sensing portion 11.

The radially inner surface 10a comprises a base portion 15 and a protrusion portion 16 protruding radially inward with respect to the base portion 15. By protruding, it is meant that the base portion defines a smooth geometrical surface roughly corresponding to the overall outer geometry of the user's limb, and that the protrusion portion 16 protrudes from this ideal geometry. The overall geometry of the base portion 15 fits that of the limb of the user. The protrusion portion 16 fits the geometry of the underlying hard tissues of the wearer's limb, and can in general be pressed facing the radial artery 6 into the anatomical recess defined between the brachioradialis muscle tendon 7, the flexor carpi radialis muscle tendon 8 and by the pronator quadratus muscle 9. The flexible film 53 is provided with high deformation capacity in order to be able to accommodate the underlying geometry of the user's limb. Hence, the radially inner portion of the sensing portion is highly flexible, has a controlled shape and stiffness so as to apply controlled pressure on the user's limb. This enables a controlled, tight, reliable and constant contact of the device with the user's limb.

According to one option, at rest (ie before wearing the device), the device may exhibit such a protrusion portion 16. According to another option, at rest, the device will not exhibit such a protruding portion, and will have a smooth surface. The protrusion will appear when the device is tensed on the user's limb, because the device will adapt to the user's limb underlying geometry. This ensures a proper wear of the device, because, however the device is worn, it ensures the protrusion to be located where it should. The user can freely move while continuing to have the device operative.

The sensing portion 11 further comprises a sensor to sense blood pressure. The sensor thus operates as a pressure sensor or pressure transducer. According to one first embodiment, the sensor can comprise a vibration sensor 17 to sense blood pressure transmitted from the user limb through the protrusion portion 16. In the present example, the vibration sensor 17 is shown comprising a sensor array comprising two individual sensors 17a and 17b. In alternative embodiments, the vibration sensor may comprise one single individual vibration sensor, or more than two individual vibration sensors, for example. Using two individual vibration sensors rather than one may improve the overall signal-to-noise ratio of the sensor 17, for an acceptable bulk. More sensors may further improve this signal-to-noise ratio but may be detrimental to the wearability of the monitoring device.

As can be seen on Fig. 4, in the present embodiment, the blood pulse is transmitted to the sensor 17 through fluid. More specifically, the monitoring device comprises a chamber 18 defined by a fluid-tight envelope 19 and filled with fluid 20. The film 53 forms part of this envelope 19, in particular the radially inward wall of the envelope 19. The fluid is for example a suitable liquid or gel, preferably incompressible, of dynamic viscosity comprised between 0.5 and 1 milliPascal.second (mPa.s), such as, for example, water, ethanol, or inert organic liquids, such as silicone oils (for example polydimethylsiloxanes). The sensor 17 is comprised within the chamber 18. Alternatively, the sensor 17 may be attached to the chamber wall so that its active detection portion is within the fluid. In the sensing portion 11, the monitoring device comprises an inner wall 13 and an opposed outer wall 14 which are part of the fluid-tight envelope 19. The inner wall 13 faces the user's limb. The radially inner surface 10a is that of the inner wall 13. The radially outer surface 10b can be that of the outer wall 14.

In use, the monitoring device is tightened on the user's limb, and the protrusion portion 16 is maintained in close contact with the user's skin facing the radial artery 6. The envelope 19 is filled with fluid so that heart-beat-induced movement of the artery is transmitted through user tissue, inner wall 14 and fluid 20 to the sensor 17. The sensor comprises a microphone adapted to detect these movements. Alternatively, the sensor comprises a hydrophone designed to detect these movements inside the fluid. Any movement detection sensor can be used. Other technologies are possible, as discussed below.

According to an embodiment, the radially outer portion 21 (and especially for example the outer wall 14) is stiff, i.e. stiffer than the radially inner portion (and especially for example the inner wall 13), in order to ensure that the monitoring device cannot be deformed in a way that the protrusion portion 16 does not any more face the radial artery 6 nor that the blood-flow-induced movement of the artery is not transmitted to the sensor 17.

The fluid thus has a double function. First, it flows so that the space between the radially outer portion and the radial artery 6 is filled with material (other than air), including anatomical structures (skin), the inner wall, which is highly flexible, and the fluid itself. Second, it conveys the deformation occurring at the artery due to blood flow to the sensor 17.

When one discusses stiffness of the inner wall 13, this in fact is the average stiffness of the inner wall 13. Indeed, the inner wall 13 may have portions of different stiffness. Hence, according to one embodiment, the radially inner portion comprises a main portion and localized portions (part of the protrusion portion may comprise one localized portion), and the localized portions are stiffer than the main portion. This helps ensuring the shape of the protrusion portion 16 as discussed above. Stiffer localized portions are for example provided at the junction between the base portion and the protrusion portion to ensure the protrusion portion maintains a protruding shape even though the constituting material of the inner wall is highly flexible.

Suitable materials for the band, and especially for the inner wall 13, include elastomers. One may for example consider natural rubber. A natural rubber with an elasticity modulus of about 1 to 20 MegaPascals (MPa) is believed suitable.

Another example comprises synthetic rubbers. A typical example could be terpolymers of ethylene, propylene and a diene component, such as EPDM. One may in particular select materials with one or more of the following properties:
maximum tensile stress: 13-20 MPa;
maximum tensile strain: 450%;
Shore A hardness: 30-90.

Another example comprises polyurethane. One may in particular select materials with one or more of the following properties:
maximum tensile stress: 70 MPa;
maximum tensile strain: 600%;
Shore A hardness: 20-80;
Elasticity modulus: 1-20 MPa, preferably 2-10 MPa.

Another example comprises silicone. The table below lists suitable silicones and their elastic modulus at 100% deformation :

| | |
|---|---|
| Silicone 30 Shore A | 0.7 |
| Silicone 70 Shore A | 3.3 |
| Nitrile 50 Shore A | 1.5 |
| Nitrile 75 Shore A | 5.8 |
| **Material** | **Module at 100% deformation (MPa)** |

Optionally, the radial inner surface 10a may be textured in order to improve the passage of air and/or liquid (sweat, ...) at the skin under the band.

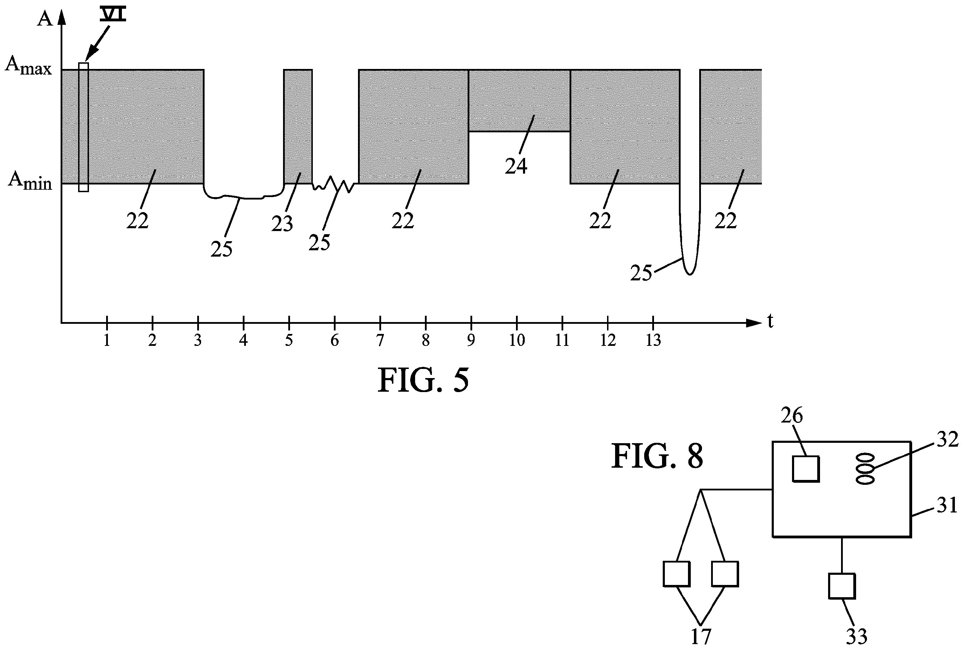

As show on Fig. 8, the monitoring device 1 further comprises an electronic control unit 31. The electronic control unit 31 is connected to the sensor(s) 17, by any suitable means, such as a wire, as shown, or wire-less. This connection can be permanent, or not permanent, in which case it may occur from time to time. The electronic control unit 31 can thus be physically part of the wristband, or can be remote. The electronic control unit 31 may comprise a processor 26 which is suitable for treating data, in particular blood-pulse related data measured by the sensor(s) 17. The electronic control unit may also comprise a memory 32 to store any data relevant to be stored within the present process.

Fig. 5 shows part of a typical signal which is obtained from a sensor 17. The signal shown on Fig. 5 represents the amplitude of the measured plethysmogram as a function of time. The time duration shown on Fig. 5 is about 15 hours. As discussed above, since the monitoring device will be worn for a long time, typically over 6 hours, and notably for more than one day, potentially at least 7 days or even 30 days, data can be measured while the monitoring device 1 is worn. As can be seen on Fig. 5, the measured signal comprises different portions, on a macroscopic scale, along the time of interest. For example, the measured signal comprises blocks 22 where the signal is similar to a periodic signal of main frequency f comprised in an interval between fₘᵢₙ=0,5 and fₘₐₓ=2 Hertz (Hz) and with amplitude comprised between Aₘᵢₙ and Aₘₐₓ, for a duration over a duration dₘᵢₙ. frequencies fₘᵢₙ and fₘₐₓ, amplitudes Aₘᵢₙ and Aₘₐₓ can be predetermined in the system, and duration dₘᵢₙ can be predetermined in the system to, for example, at least one hour. The typical measured signal may comprise other parts which do not respect these conditions. For example, on Fig. 5, a block 23 is illustrated which does not match the condition of the duration being over the predetermined duration dₘᵢₙ. A block 24 is illustrated, where the minimal amplitude of the signal is over the predetermined Aₘᵢₙ by a significant value over a pre-determined threshold t. Some parts 25 of the signal are not periodic. Other examples can be encountered.

Hence, the signal as such, such as shown on Fig. 5, does not enable to obtain a relevant blood-pulse related parameter. The processor 26 generates high-quality data from the measured signal. This is achieved by filtering out low-quality data from said signal. The processor 26 determines whether a part of the signal is sufficiently relevant for further processing. For example, the processor 26 will make use of pre-determined parameters dₘᵢₙ. Aₘᵢₙ and Aₘₐₓ, fₘᵢₙ and fₘₐₓ to identify if a part of the signal is to be filtered out.

By this processing, the processor 26 can for example filter out:
- parts 25 of the signal because they do not respect the condition of the main frequency being between fₘᵢₙ and fₘₐₓ,
- block 24 of the signal, because it does not respect the condition of the minimal amplitude being close enough to Aₘᵢₙ,
- block 23 of the signal, because it does not respect the condition of the duration being over a pre-determined threshold.

in particular, some parts of the signal can be filtered out based on information provided by another system 33 of the monitoring device 1. For example, the other system 33 includes an inertia measurement unit (accelerometer, goniometer, ...). The processor 26 is configured to determine a risk that a part of the signal be of low quality based on information provided by the other system 33. For example, if data provided by the other system 33 enables to determine that the user is performing movements which may cause motion artefacts in the measured signal, this information is used to filter out that part of the measured signal. The other system 33 may thus be part of the monitoring device 1, being for example part of the wristband, and connected to the electronic control unit 31 is any suitable way, such as the sensor(s) 17 is/are.

It is believed that parts 24 and 25 of the signal might not be relevant for later treatment for various reasons. A possible reason includes user motion artifacts, since the user is moving while wearing the monitoring device 1. Despite all due care, it can not be prevented that, from time to time, some motion of the monitoring device 1 with respect to the user will cause measurement of signals irrelevant for a proper measurement of a relevant blood-pulse related parameter. Under the invention, the monitoring device will be worn during long periods of time, typically over 6 hours, probably over 24 hours or even many days. The above process enables to filter out measured data which may be not reliable for later treatment. This is based on the belief that, upon such a long duration, at least part of the signal will be relevant enough for future treatment. This is also based on the belief that it would be more relevant not to provide any blood-pulse related parameter than an irrelevant parameter, if one is not confident enough in the quality of the measurement. The processor 26 may also generate an alarm or other messaging signal when it determines that not relevant enough data has been recorded over a period of time exceeding a predetermined threshold. This could prompt the user to check whether the monitoring device is properly worn and, if necessary, adapt the position of the monitoring device if this was dislodged due to an unwanted motion.

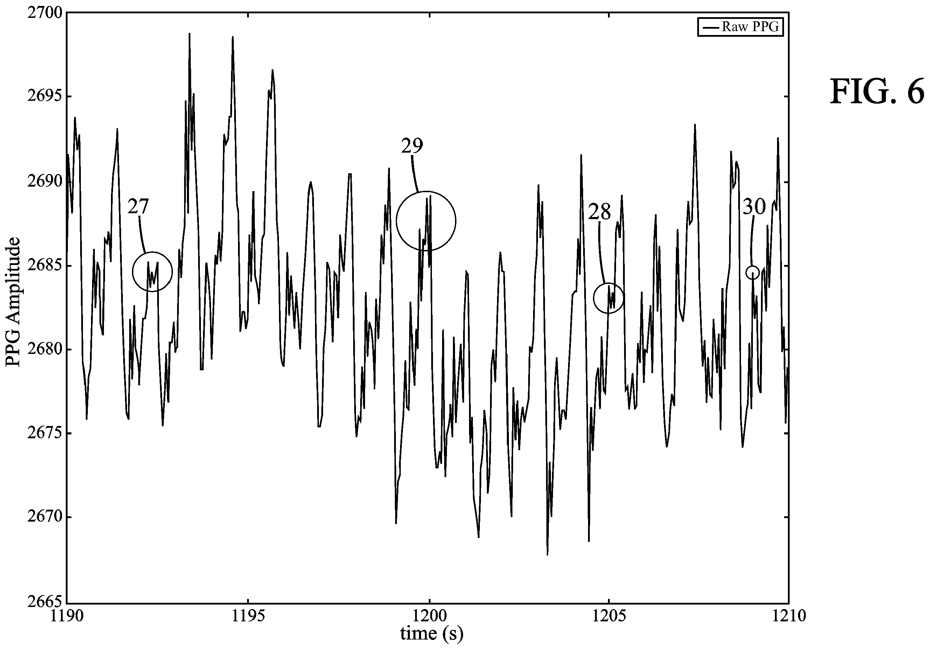

Fig. 6 shows part of a typical signal which is obtained from a sensor 17 and was not filtered out during the above-described initial stage. The signal shown on Fig. 6 represents the amplitude of the measured plethysmogram as a function of time. The time lapse shown on Fig. 6 is 20 seconds, extending from time 1190s to time 1210s. As can be seen from Fig. 6, this signal features about 19 pulses of varying signal shapes.

One relevant parameter when determining a blood-pulse related parameter involves a variability of a RR signal of the plethysmogram, where "R" designates the time of a period where the amplitude reaches the maximum for the period, and "RR" designates the time duration between two subsequent Rs. The "R" peak is part of the R wave of an electro cardiogram, especially of the QRS complex of the electrocardiogram. As can be seen from Fig. 6, it is not always straightforward to identify the proper Rs to be used, especially when the data is measured by a wearable device.

Issues when identifying the proper Rs include:
- Identifying whether part of the signal includes a peak to be considered -compare for example parts 27 and 28 of the signal, which have similar amplitudes, but do not share similar relationships to adjacent peaks,
- Identifying which part of the signal corresponds to the relevant peak - see for example part 29 which comprises 2, and potentially 4 candidates for peak R.

An exemplary treatment is described below. Considering an embodiment where parts 25, 23 and 24 of the signal were filtered out during a prior treatment, the exemplary treatment described below can be performed only on all blocks 22.

To start with, a pre-processing treatment may be applied to the data. This pre-processing may include a low pass filter. The low-pass filter may be useful to remove signal noise from the data. This pre-processing may include a high pass filter. The high-pass filter may be useful to remove the continuous part of the measured signal. According to one embodiment, the pre-processing treatment may include both a low-pass filter and a high-pass filter.

The signal is then processed in order to be identifiable with blood pulse. For example, the signal is considered as a sum of periodic signals of different frequencies, and the part of the signal corresponding to frequencies outside a pre-determined frequency range are filtered out. The pre-determined frequency range corresponds to that of blood pulse. For example, it can be set to [f1; f2], where f1 is between 0.5 and 0.8 Hz and f2 is between 2 and 3 Hz. f1 and f2 can be tailored according to requirements. For example, they may be user specific.

According to one embodiment, this can be performed using empirical mode decomposition. This involves composing the signal as a sum of intrinsic mode functions of constant frequencies and amplitudes. These intrinsic mode functions might be selected by the processor 26 from the signal itself. The intrinsic mode functions having a frequency outside the [f1; f2] range and those having an amplitude below a pre-determined threshold are filtered out. This treatment step provides a signal which is close to a blood-pulse signal of the user. In particular, only the main empirical mode is kept for further processing.

The Rs of the signal are determined. In particular, the processor 26 determines local maxima in the signal along time. For example, the Rs of the signal are determined as a selection of the peaks (local maxima) of the signal, notably after a filtration treatment as described above. According to one embodiment, all peaks are selected. According to another embodiment, a part only of the peaks are selected. According to one embodiment, peaks are selected based on criteria involving the amplitude of the peak and/or the distance of the peak with neighboring peaks. For example, at this stage, although the above-described treatment did not enable to filter out a peak corresponding to part 30 of the signal on Fig. 6, this peak is filtered out at this stage as not being a R to be taken into account for further processing.

For example, the peaks are selected by minimizing a cost function tending to obtain the most regular time duration between adjacent peaks. This is in particular needed when a group of subsequent peaks of the signal are closer to one another than R peaks should be. In this case, the selected peaks are those who enable to have the most regular time interval between the selected peaks.

If subsequent peaks are further away from one another than R peaks should be, one may split the signal in two parts separated by the long gap between the two adjacent peaks, and apply any following treatment independently on each part of the signal.

One or more of the above-described processes enable to obtain data of higher quality than the raw originally measured data. Data of higher quality may enable to measure a blood-pulse related parameter of the user based on measurement from a wearable device which may be sufficiently relevant to be used in a medical environment.

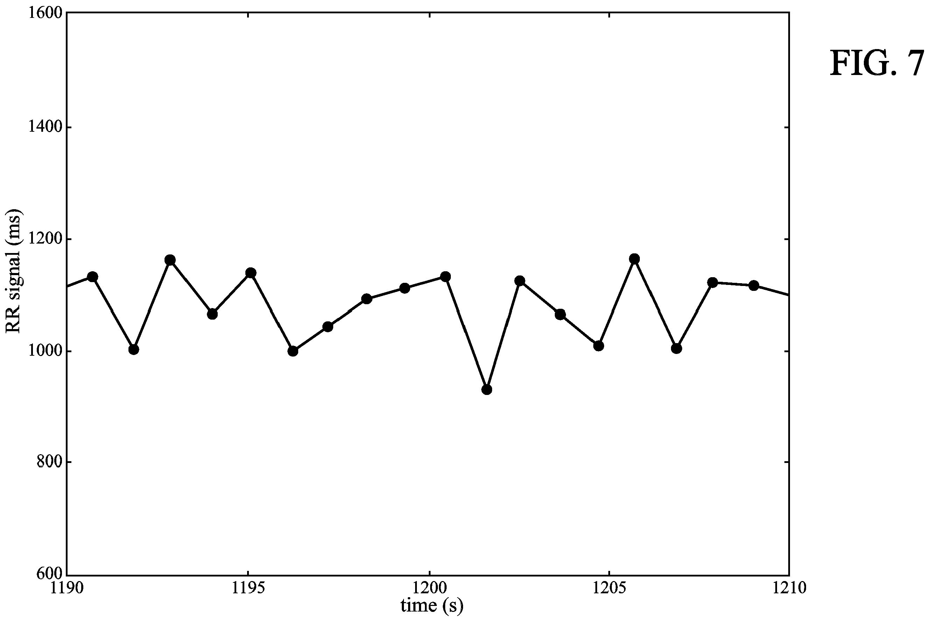

The processor 26 determines the variablility of the RR signal. This may involve explicitly determining the RR signal. In particular, this can be done independently for each separate part of the signal, if the above-treatment led into filtering out part of the originally measured data. The RR signal may be determined based on the Rs determined according to the above-described process. Fig. 7 shows the RR signal, in milli-second, along time (in seconds), for the above-described signal submitted to the above-described signal treatment. The RR signal at time t is determined as the time difference between the time of the R peak and the time of the previous R peak. In order to obtain a relevant RR signal, it is important to be able to define the R peaks with a high probability of likelihood. A false R would affect two neighboring points of the RR signal. The above-described method is believed to be an example of a relevant method to obtain a relevant RR signal.

A blood pulse-related parameter of the user can be determined based on the RR signal for the user. Notably, one could use the method of determining the RR signal of the user as described above, which is believed to be able to provide accurate R data for the user, based on a long data acquisition by a wearable device.

One typical parameter of relevancy is believed to be related to the standard deviation of the RR signal. More specifically, a parameter of relevancy may be obtained from the evolution of the standard deviation of the RR signal along time. An example of a process for determining this parameter of relevancy will be described below.

The processor 26 determines a standard deviation SDNN of the RR signal as a function of time. One may use the RR signal as obtained from the above-described process, and as shown on Fig. 7. The processor 26 determines the SDNN as a function of time. This determination is performed for a pre-determined time interval duration D. For example, D can be selected as being 1 to 10 minutes, which appears a relevant interval for the RR signal. The SDNN of the RR signal for time t is thus determined as the standard deviation of the RR signal between the time t-D/2 and the time t+D/2. Alternately, time may be split in intervals [tᵢ; tᵢ₊₁[, for example regular intervals and, for all times t in a given interval [tᵢ; tᵢ₊₁[, one may use the same part of the signal RR between the time t₀-D/2 and the time t₀+D/2, where t₀ is a selected value in the interval, in particular the average value of the interval [tᵢ; tᵢ₊₁[.

One typical parameter of relevancy is believed to be related to the high frequency and/or low frequency spectral density of the RR signal. An example of a process for determining these parameters of relevancy will be described below.

According to some embodiments (and in particular according to the above-described embodiment), the RR signal is not regularly sampled. This means that the time interval between two measurement points varies with time. The low frequency spectral density and/or the high frequency spectral density of the RR signal can be determined by the processor 26 without re-sampling or interpoling the RR signal, so as to limit the introduction of bias. For the determination of the blood-pulse related parameter, the processor 26 may apply the Lomb-Scargle treatment to the RR signal. Unlike Fourier transform, this treatment enables to transform the unregularly-sampled signal in the frequency domain without interpolation bias. In the present case, this treatment can be applied to a part of the signal of a given duration D, typically between 1 and 10 minutes. This is assigned to a time t selected within the time interval, for example the average time value t₀ of the interval. One thus obtains, for t₀, a signal (in dB) as a function of the frequency of the RR signal. This is for example used to determine the low frequency band and/or the high frequency band of the RR signal. The low frequency band may be the part of this signal between pre-determined low frequencies f₁ and f₂. For example, f₁=0.04 Hz. For example, f₂=0.15 Hz. The high frequency band may be the part of this signal between pre-determined high frequencies f₃ and f₄. For example, f₃=0.15 Hz. For example, f₄=0.4 Hz. f₃ may be equal to f₂. However, according to other embodiments, f₃ may be greater than f₂. According to other embodiments, f₃ may be lower than f₂. The spectral density of the signal in a given frequency band may be obtained as the integral of the signal in that frequency band. The processor may for example determine the spectral density for the low frequencies, and/or the spectral density for the high frequencies. The spectral density for the low frequencies is believed to be a parameter related to stress. The spectral density for the high frequencies is believed to be a parameter related to recuperation from stress. A parameter of interest is believed to be a parameter function of the low frequency spectral density and/or the high frequency spectral density of the RR signal, such as a ratio of the low frequency spectral density to the high frequency spectral density of the RR signal.

The above parameters are thus obtained for time t₀. By repeating the above treatment for other times, one may obtain an evolution along time of this parameter. This evolution with time may be a relevant parameter.

In particular, the evolution with time of a ratio of the low frequency spectral density to the high frequency spectral density of the RR signal is believed to be a relevant blood-pulse related parameter. This parameter can for example be used for the assessment of a mental suffering such as depression. This parameter can for example be used as the time-dependent physiological parameter of WO 2016/151,135 by the applicant, which is hereby incorporated by reference in its entirety for all purposes. As taught in WO 2016/151,135, this time-dependent physiological parameter may be associated with a time-dependent behavioural parameter of the user. In the present context, a typical example of a time-dependent behavioural parameter of the user would for example be that the user is sleeping, and in particular in phase of deep sleep. This behavioural parameter may be determined by any suitable method, such as by using an accelerometer, a clock, and/or necessary sensors, in particular inside the monitoring device 1. Other index of the sympatho-vagal balances can be determined under the above-described method.

A parameter determined using the above-described method may be used in different ways. One possible way to use the parameter is to use it in a predictive way, in order to obtain information which may be related to a risk that the user may be on the way to mental suffering. One possible way would be to compare the measured parameter with measurements performed before a traumatic event. This would be possible notably if one could start measurement before a traumatic event, which might be the case for example for some jobs such as fireman, policeman, military, .... Another way may be to compare the results of the measurements for the user to a database of measurements of other users, and determine there a pattern index representative of the user's likeliness of exhibiting a pattern of mental suffering.

While one example of a monitoring device has been described in particular, the above-described method could be applied to blood-pulse related data obtained using other suitable monitoring devices, provided this data is measured for users over a sufficiently long period of time, preferably during their daily normal activity. One may use any kind of suitable plethysmograph, and in particular photoplethysmographs. Monitoring the users during their normal daily activity is an important feature for the assessment of mental suffering under WO 2016/151,135.

**References :**

| | | |
|---|---|---|
| Monitoring device 1 | radially outer surface 10b | blocks 22, 23, 24 |
| Radius 2 | sensing portion 11 | part 25 |
| Ulna 3 | attachment portion 12 | processor 26 |
| Skin 4 | base portion 15 | parts 27-30 of the signal |
| ulnar artery 5 | protrusion portion 16 | electronic control unit 31 |
| radial artery 6 | vibration sensor 17 | memory 32 |
| brachioradialis muscle tendon 7 | individual sensors 17a, 17b | other system 33 |
| flexor carpi radialis muscle | chamber 18 | flexible film 53 |
| tendon 8 | fluid-tight envelope 19 | |
| pronator quadratus muscle 9 | fluid 20 | |
| radially inner surface 10a | radially outer portion 21 | |

## Claims

1. A method to measure a blood pulse-related parameter of a user, comprising:
- measuring, for a long period of time, typically over 6 hours, blood-pulse related data of a user,
- generating high-quality data from said blood-pulse related data by filtering out low-quality data from said blood-pulse related data,
- determining said blood pulse-related parameter from said high-quality data.

2. A method according to claim 1, wherein generating high-quality data comprises :
- determining at least one part of said blood-pulse related data comprising user motion artifacts, and
- removing said at least one part from said blood-pulse related data.

3. A method according to claims 1 or 2, wherein generating high-quality data comprises :
- determining at least two parts of said blood-pulse related data comprising low-quality data,
- when the time duration between said two parts is lower than a predetermined threshold, filtering the part of said blood-pulse related data between said two parts out of said blood-pulse related data.

4. A method according to claim 3, wherein determining the blood-pulse related parameter involves determining a variability of a RR signal of a plethysmogram, where "R" designates the time of a period where the amplitude reaches the maximum for the period, and "RR" designates the time duration between two subsequent Rs.

5. A method according to claim 4, wherein determining the variability of a RR signal of the plethysmogram involves determining a RR signal of the plethysmogram.

6. A method according to claim 5, wherein determining a RR signal of the plethysmogram involves determining Rs from said high-quality data.

7. A method according to claim 6, wherein determining Rs comprises determining local maxima in said high-quality data along time, and selecting, among said local maxima, selected local maxima having values in a pre-determined window.

8. A method according to claim 7, wherein selecting said local maxima involves using a cost function tending to obtain the most regular time duration between adjacent local maxima.

9. A method according to any of claims 4 to 8, wherein determining the variability of a RR signal of the plethysmogram involves determining the variability for each part of the high quality data.

10. A method according to any of claims 4 to 9, wherein determining the variability of a RR signal of the plethysmogram involves determining an evolution over time of a standard deviation of the RR signal within a time window.

11. A method according to any of claims 4 to 10, wherein determining the variability of a RR signal of the plethysmogram involves determining an evolution over time of a parameter of the spectral density of the RR signal within a time window.

12. A method according to claim 11, wherein said parameter of the spectral density of the RR signal involves a ratio of a low frequency spectral density to a high frequency spectral density of the RR signal within said time window.

13. A method according to any of claims 11 to 12, wherein a spectral density of the RR signal is determined using a transform taking into account irregulary-sampled data without interpolation.

14. Device to measure a blood pulse-related parameter of a user, comprising:
- at least a sensor (17) measuring, for a long period of time, typically over 6 hours, blood-pulse related data of a user,
- a processor (26) generating high-quality data from said blood-pulse related data by filtering out low-quality data from said blood-pulse related data,
Wherein said processor is further configured to determine said blood pulse-related parameter from said high-quality data.

15. A computer program comprising instructions which, when the program is executed by a computer, cause the computer to carry out the method of any of claims 1 to 13.
